# EUROPÄISCHE PATENTSCHRIFT

(11) **EP 0 861 668 B1**
(45) Veröffentlichungstag und Bekanntmachung des Hinweises auf die Patenterteilung: **09.02.2005**
(21) Anmeldenummer: 97103068.9
(22) Anmeldetag: 26.02.1997
(51) Int. Cl.: A61M 1/00

(54) **Einrichtung zum Absaugen von Flüssigkeiten**
Fluid suction device
Dispositif de succion de fluides

(43) Veröffentlichungstag der Anmeldung: 02.09.1998
(73) Patentinhaber: Medela AG, 6340 Baar (CH)
(72) Erfinder: Greter, Andy, 6312 Steinhausen (CH)
(74) Vertreter: Clerc, Natalia

(56) Entgegenhaltungen:
- EP-A- 0 378 296
- WO-A-94/14045
- DE-A- 3 500 538
- FR-A- 2 716 806
- US-A- 3 680 560
- US-A- 4 821 896

## Beschreibung

Die vorliegende Erfindung betrifft eine Einrichtung zum Absaugen von Flüssigkeiten, insbesondere von Körperflüssigkeiten im Rahmen medizinischer Behandlungen, bei welcher die Flüssigkeit mittels Unterdruck über eine Absaugleitung in einen Auffangbehälter geleitet wird, wobei der Auffangbehälter als in einem starren Aussenbehälter mit luftdicht verschliessbarem Deckel untergebrachtem Einwegbeutel ausgebildet ist.

Im medizinischen Bereich sind für das Absaugen von Körperflüssigkeiten heute die verschiedensten Systeme bzw. Methoden bekannt. In all jenen Fällen, wo die Körperflüssigkeiten durch Anwendung von Unterdruck abzusaugen und aufzufangen sind, kann dies z.B. dadurch geschehen, dass ein Auffangbehälter aus starrem Material mit einem Deckel versehen wird, in welchen einerseits die Absaugleitung führt und andererseits ein Anschluss zum Aufbau eines Unterdruckes vorgesehen ist, wobei dieser Anschluss zu einer Saugquelle (Saugpumpe) führt. Das Problem bei diesem System liegt darin, dass zur Entsorgung der aufgefangenen Flüssigkeiten der Behälter als Ganzes zu transportieren ist und nach dem Entleeren des Inhaltes einer sorgfältigen Reinigung zu unterziehen ist. Dieses System entspricht nach neuesten Erkenntnissen in gewissen Fällen den Anforderungen an die Hygiene nicht mehr. Um dieses Problem zu lösen, wurde vorgeschlagen, im Innern des starren Behälters einen Einwegbeutel unterzubringen, welcher allein zum Auffangen der abzusaugenden Flüssigkeiten dient. Ein solcherart mit Flüssigkeit gefüllter Beutel kann zur Entsorgung nach Abnehmen des Behälterdeckels entfernt und der eigentlichen Entsorgung zugeführt werden, während der starre Behälter in der Regel bereits mit einem neuen Beutel ausgerüstet werden kann. Das Problem bei diesem System liegt darin, dass zwei verschiedene Unterdruck-Leistungen (Vakuum-Leistungen) erforderlich sind, nämlich die eine, um den Beutel aufzuweiten und diesen gegen die BehälterInnenwand anzulegen, und ein zweiter Anschluss, welcher ins Beutelinnere führt, um nach dessen Aufweitung im Beutelinnern den erforderlichen Unterdruck zu erzeugen. Dabei ist immer darauf zu achten, dass der Unterdruck im den Beutel umgebenden Raum höher sein muss als jener im Innern des Beutels, da sonst die Gefahr besteht, dass der Beutel wieder zusammenklappt und eine weitere Füllung nicht mehr möglich ist. Ganz abgesehen davon, ist eine solche Konstruktion aufwendig und somit teuer.

US-A-3'680'560 offenbart eine Drainage-Einrichtung, bei welcher ein in einem starren Aussenbehälter angeordneter Auffangbehälter mit einer ersten Öffnung für eine Absaugleitung und mit einer mittels einer gasdurchlässigen Membran verschlossenen zweiten Öffnung versehen ist. Bei Beginn der Saugwirkung wird zuerst der Raum zwischen dem Auffangbehälter und dem Aussenbehälter evakuiert und dadurch der Auffangbehälter expandiert. Anschliessend setzt die Saugwirkung durch die Membran hindurch im Beutelinnern und damit in der Absaugleitung ein. Die Befestigung des Auffangbehälters innerhalb des Aussenbehälters erfolgt dadurch, dass der Auffangbehälter mit einem Anschlussstutzen direkt an die Saugleitung angekuppelt wird.

Eine ähnliche Lösung ist in WO 94/14045 offenbart. Hier ist der Auffangbehälter als Einwegbeutel ausgebildet und mit einem starren Kopfteil versehen. Dieser starre Kopfteil bildet zugleich den Deckel des Aussenbehälters.

Aufgabe der vorliegenden Erfindung war es somit, eine Einrichtung zu schaffen, mit welcher bei einfachster Konstruktion das Auffangen von Flüssigkeiten in flexiblen Auffangbeuteln problemlos möglich ist. Dabei soll sich die Grundausrüstung zudem auch für ein System eignen, bei welchem die abzusaugende Flüssigkeit durch den starren Auffangbehälter aufgenommen werden kann (für den Fall von problemlosen Flüssigkeiten).

Die Aufgabe wird nun bei einer Einrichtung der eingangs definierten Art erfindungsgemäss durch die Merkmale gelöst, wie sie im kennzeichnenden Teil von Anspruch 1 definiert sind.

Dank der erfindungsgemässen Konstruktion wird es mit einer einzigen Unterdruckleitung möglich, zuerst den flexiblen Behälter im Innern des luftdicht abgeschlossenen Auffangbehälters aufzuweiten und in dieser Stellung zu halten und erst nach dem Aufweiten die Saugwirkung ins Innere des Beutels zu verlegen. Beim aufgeweiteten Beutel, welcher durch den angelegten Unterdruck immer in diesem erforderlichen Zustand gehalten wird, sorgt die gasdurchlässige Membran dafür, dass durch diese keine Flüssigkeit austreten kann (Sperrmembran), jedoch Luft zwecks Erzeugung des erforderten Unterdruckes abgesaugt werden kann. Durch diese einfache Massnahme, welche sich durch das starre Kopfteil am Beutel realisieren lässt, entsteht eine ausserordentlich einfach aufgebaute Einrichtung, welche auch dann benutzt werden kann, wenn keine Einwegbeutel zum Einsatz gelangen. In diesem Fall wird der Innenraum des starren Auffangbehälters evakuiert und die Flüssigkeit direkt in diesen Behälter abgesaugt. Die vorgeschlagene erfindungsgemässe Einrichtung lässt sich somit universell anwenden.

Besondere Ausführungsformen der erfindungsgemässen Einrichtung sind in den abhängigen Ansprüchen 2 bis 4 definiert.

Ebenfalls Gegenstand der Erfindung ist ein Einwegbeutel zur Aufnahme von abgesaugter Flüssigkeit, welcher sich insbesondere zur Verwendung in der Einrichtung nach den Ansprüchen 1 bis 4 eignet, und welchersich durch die Merkmale gemäss kennzeichnendem Teil von Anspruch 5 auszeichnet.

Eine besondere Ausführungsform dieses Einwegbeutels ist in Anspruch 6 definiert.

Es sei nochmals wiederholt, dass beim Einsatz von Einwegbeuteln dafür Sorge getragen werden muss, dass beim Eintritt der Saugwirkung die Beutelwände nicht einfach zusammenklappen, sondern dass der Beutel zuerst in Richtung der Wand des starren Auffangbehälters aufgeweitet bzw. gegen die Behälterwände angelegt werden muss. Das war bisher nur unter Einrichtung von zwei verschiedenen Vakuumleitungen möglich, wobei die eine zum Aufweiten des Beutels diente, die andere zum Erzeugen des Vakuums im Beutelinnern. Nachteilig an diesem System war einerseits der komplexe Aufbau, und zudem konnte die Wirkung nicht immer gewährleistet werden, ohne die Einrichtung nochmals zu komplizieren.

Die Erfindung wird anschliessend anhand eines in der Zeichnung dargestellten Ausführungsbeispiels noch etwas näher erläutert. Es zeigen:
- Fig. 1: Einen Vertikalschnitt durch einen starren Auffangbehälter, in welchen ein flexibler Einwegbeutel mit starrem Kopfteil eingesetzt ist;
- Fig. 2: rein schematisch eine Ansicht von unten auf die Unterseite des Behälter-Deckels mit eingesetztem Einwegbeutel und
- Fig. 3: eine perspektivische Ansicht des starren Kopfteils eines Einwegbeutels gemäss Fig. 1.

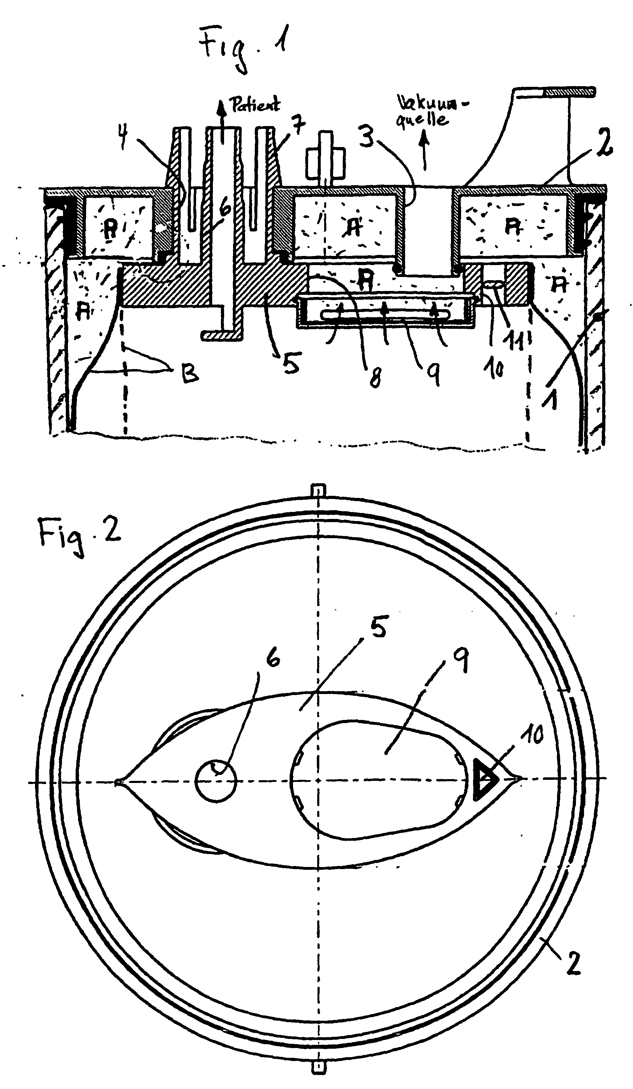

Figur 1 der Zeichnung zeigt rein schematisch eine Einrichtung nach der Erfindung. Dabei ist auf einen starren Behälter 1 ein Deckel 2 so aufgesetzt, dass ein luftdichter Verschluss entsteht. Dies wird durch entsprechende Dichtungen zwischen Behälter 1 und Deckel 2 realisiert. Im Deckel 2 selbst ist eine Anschlussöffnung 3 für den Anschluss einer Verbindungsleitung zu einer Saugquelle (Saugpumpe, nicht dargestellt) vorgesehen, in welche die Verbindungsleitung ebenfalls luftdicht einsteckbar oder anderweitig anschliessbar ist. Der Behälterdeckel 2 weist ferner eine Anschlussöffnung 4 zum Anbringen einer zum Patienten führenden Absaugleitung auf (nicht dargestellt).

Wie die Zeichnung zeigt, ist von der Unterseite des Behälterdeckels her ein starres Kopfteil 5 eines flexiblen Einwegbeutels B befestigt. Das starre Kopfteil 5 besteht aus einer schiffchenförmigen Platte, von welcher einerseits ein Anschlussnippel 6 durch die Behälterdeckel-Öffnung 4 nach oben führt und welche von federnden Rastklinken 7 umgeben ist. Der Nippel 6 dient zum Aufstecken der zum Patienten führenden Absaugleitung. Zwischen der Kopfplatte 5 des Beutels B und dem Behälterdeckel 2 ist eine Dichtung vorgesehen, um die Verbindung luftdicht zu gestalten. Die Rastklinken 7 lassen sich durch die Deckelöffnung 4 hindurchführen und spreizen dann in die Halteposition aus, wie dies aus Fig. 1 hervorgeht. Damit ist die starre Platte 5 des Einwegbeutels fest auf der Unterseite des Deckels angebracht. Bei angeschlossener Absaugleitung und Verbindungsleitung zur Saugquelle ist klar ersichtlich, dass der Innenraum des Behälters 1 gegenüber der Umgebung völlig abgedichtet ist.

In der Kopfplatte 5 des Beutels ist eine weitere Öffnung 8 vorgesehen, in welcher eine Membran 9 festgehalten ist. Diese Membran 9 ist bezüglich Flüssigkeiten undurchlässig, jedoch unter Überwindung eines geringen Widerstandes für Gas bzw. Luft durchlässig.

In der Kopfplatte 5 des Beutels B ist eine weitere Durchgangsöffnung 10 vorgesehen, welche im Normalfall durch ein ausbrechbares Element 11 verschlossen ist. Diese Öffnung könnte dazu dienen, bei Entleerung eines Beutels B als Entlüftung zu dienen, um das Ausströmen der aufgefangenen Flüssigkeit durch den Nippel 6 hindurch zu erleichtern, falls dies notwendig wäre. In der Regel wird allerdings eine Entleerung durch Druck auf die Beutelwand ausreichen.

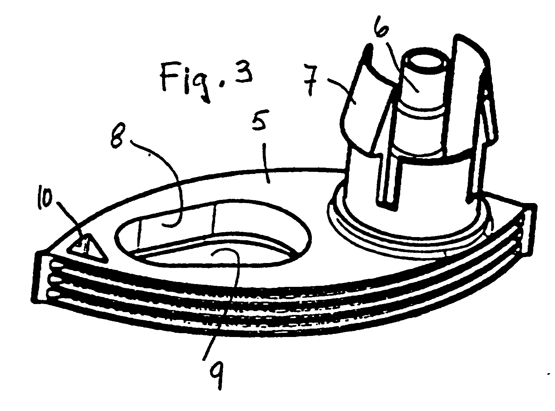

Zur allgemeinen Illustration zeigt Fig. 3 noch eine perspektivische Darstellung des starren Kopfteils 5 eines Einwegbeutels B.

Wenn nun mit auf der Unterseite des Deckels 2 angebrachtem Beutel (mit im Deckel festgehaltener bzw. festgeklemmter Kopfplatte 5 des Beutels B, angeschlossener, zur Vakuumquelle führender Leitung und angeschlossener Patientenleitung) die Vakuumquelle in Betrieb genommen wird, d.h. durch den Anschluss hindurch eine Saugwirkung erzeugt wird, wird zuerst am oberen Ende des Behälters, in den Räumen A die Luft evakuiert und damit der Beutel B mit seinen Seitenwänden in Richtung zu den Behälterwänden 1 aufgeweitet bzw. an diese angelegt, danach wird auch die Luft durch die Membrane 9 hindurch im Beutelinnern abgesaugt und dabei ein Unterdruck erzeugt, welcher wiederum dafür sorgt, dass die abzusaugende Flüssigkeit über den Patientenanschluss ins Innere des Beutels B gelangt.

Durch die erfindungsgemässe einfache Konstruktion, insbesondere des starren Kopfteils 5 des Einwegbeutels, ist eine Einrichtung geschaffen, welche mit einem minimalen Konstruktionsaufwand das Absaugen von Flüssigkeiten in einen Einwegbeutel ermöglicht. Gleichzeitig eignet sich diese Einrichtung auch für den normalen Einsatz, d.h. das Absaugen von Flüssigkeiten direkt in den starren Behälter 1. Dazu kann entweder derselbe Deckel 2 verwendet werden oder aber ein separater Deckel vorgesehen sein, welcher einen Anschluss für eine Absaugleitung und einen Anschluss für eine Vakuumquelle aufweist.

## Patentansprüche

1. Einrichtung zum Absaugen von Flüssigkeiten, insbesondere von Körperflüssigkeiten im Rahmen medizinischer Behandlungen, bei welcher die Flüssigkeit mittels Unterdruck über eine Absaugleitung in einen Auffangbehälter (B) geleitet wird, wobei die Einrichtung den Auffangbehälter (B) und einen starren Aussenbehälter (1) aufweist, wobei der Auffangbehälter (B) als im Aussenbehälter (1) untergebrachtem Einwegbeutel (B) ausgebildet ist, wobei der Einwegbeutel (B) mit einem starren Kopfteil (5) ausgerüstet ist und wobei der starre Kopfteil (5) einerseits einen Einlass aufweist und andererseits eine mit einer gasdurchlässigen Membran (9) verschlossene Hilfsöffnung (8) aufweist, wobei bei Beginn der Saugwirkung zuerst der Raum (A) zwischen Beutelaussenwand und Innenwand des Aussenbehälters (1) evakuiert und damit die flexible Beutelwand in Richtung der Behälterwand angelegt wird und erst danach die Saugwirkung durch die Membran (9) hindurch im Beutelinnern und damit in der Absaugleitung einsetzt,
**dadurch gekennzeichnet,**
**dass** zusätzlich zum starren Kopfteil (5) ein den Aussenbehälter (1) luftdicht verschliessbarer Deckel (2) vorhanden ist, welcher neben einem Anschluss (4) für die Absaugleitung lediglich mit einem Anschluss (3) zur Verbindung mit einer Saugquelle ausgerüstet ist und
**dass** der starre Kopfteil (5) von der Unterseite her an den Behälterdeckel (2) anschliessbar und von letzterem gehalten ist, wobei der Einlass luftdicht mit dem Anschluss (4) für die Absaugleitung kuppelbar ist und wobei der Verbindungsanschluss (3) des Deckels (2) zur Saugquelle frei in den oberhalb des starren Kopfteils (5) liegenden Abschnitt des Aussenbehälters (1) mündet und
**dass** Halte- und Kupplungsglieder in Form von federnden Rastklinken (7) vorhanden sind zur Kupplung des Kopfteils (5) des Beutels (B) mit dem Behälterdeckel (2), wobei diese Halte- und Kupplungsglieder den Einlass umgeben.

2. Einrichtung nach Anspruch 1, **dadurch gekennzeichnet, dass** der Absaugleitungseinlass am Kopfteil (5) des Beutels (B) als Anschlussnippel (6) ausgebildet ist, welcher von den konzentrisch dazu angeordneten Rastklinken (7) umgeben ist.

3. Einrichtung nach Anspruch 2, **dadurch gekennzeichnet, dass** die Rastklinken (7) nach oben abstehen und dass zwischen der Nippelaussenwand und Innenseite der Rastklinken (7) ein nach oben offener Ringraum zum Einstecken der Absaugleitung vorgesehen ist.

4. Einrichtung nach einem der Ansprüche 1 bis 3, **dadurch gekennzeichnet, dass** im starren Kopfteil (5) des Beutels (B) ein zusätzlicher Durchgang (10) vorgesehen ist, welcher mit einem vorzugsweise ausbrechbaren Verschluss (11) versehen ist, um zur Beutelentleerung eine Entlüftungsöffnung zu bilden.

5. Einwegbeutel zur Aufnahme von abzusaugender Flüssigkeit und zur Verwendung in einer Einrichtung gemäss einem der Ansprüche 1 bis 4, wobei er mit einer Kopfplatte (5) aus starrem Material ausgerüstet ist, wobei einerseits ein nach oben abstehender Anschluss für eine Absaugleitung vorgesehen ist und andererseits eine mit einer flüssigkeitsperrenden, aber gasdurchlässigen Membran (9) verschlossene Öffnung (8) vorgesehen ist, durch welche im Beutelinnern ein Unterdruck erzeugbar ist, **dadurch gekennzeichnet, dass** der Anschluss für die Absaugleitung gleichzeitig als Befestigungsvorrichtung ausgebildet ist, um den Beutel (B) von der Unterseite her an einem Deckel (2) eines starren Auffangbehälters (1) zu befestigen, wobei Halte- und Kupplungsglieder in Form von federnden Rastklinken (7) vorhanden sind zur Kupplung der Kopfplatte (5) des Beutels (B) mit dem Deckel (2).

6. Einwegbeutel nach Anspruch 5, **dadurch gekennzeichnet, dass** in der Kopfplatte (5) ein zusätzlicher, mit einem vorzugsweise ausbrechbaren Verschluss (11) versehener, eine Entlüftungsöffnung bildender Durchgang vorgesehen ist.

## Claims

1. Device for suctioning off of fluids, in particular of body fluids in the context of medical treatments, in which device the fluid is conveyed by means of underpressure into a collection container (B) via a suction line, the device having the collection container (B) and a rigid outer container (I), the collection container (B) being designed as a disposable bag (B) accommodated in the outer container (1), the disposable bag (B) being equipped with a rigid head part (5), and the rigid head part (5) having, on the one hand, an inlet and, on the other hand, an auxiliary opening (8) sealed by a gas-permeable membrane (9), and, at the start of the suctioning action, the space (A) between the outside wall of the bag and the inside wall of the outer container (1) is first evacuated, the flexible bag wall thus being drawn in the direction of the container wall, and only after this is the suctioning action established through the membrane (9) in the inside of the bag and thus in the suction line, **characterized in that**, in addition to the rigid head part (5), a lid (2) is provided which can close off the outer container (1) in an airtight manner and which, apart from having a connector (4) for the suction line, is equipped only with a connector (3) for connection to a suction source, and **in that** the rigid head part (5) can be attached from underneath to the container lid (2) and is held by the latter, the inlet being able to be coupled in an airtight manner to the connector (4) for the suction line, and the connector (3) of the lid (2) to the suction source opening freely into the part of the outer container (1) lying above the rigid head part (5), and
**in that** holding and coupling members in the form of resilient catches (7) are provided for coupling the head part (5) of the bag (B) to the container lid (2), these holding and coupling members surrounding the inlet.

2. Device according to Claim 1, **characterized in that** the suction line inlet on the head part (5) of the bag (B) is designed as a connector nipple (6) which is surrounded by the catches (7) arranged concentrically with respect to it.

3. Device according to Claim 2, **characterized in that** the catches (7) protrude upwards, and **in that**, between the outside wall of the nipple and the inside of the catches (7), an annular space is provided which is open to the top and serves for insertion of the suction line.

4. Device according to one of Claims 1 to 3, **characterized in that**, in the rigid head part (5) of the bag (B), an additional passage (10) is provided which is equipped with a preferably breakable seal (11) in order to form an air vent for emptying the bag.

5. Disposable bag for receiving suctioned-off fluid and for use in a device according to one of Claims 1 to 4, said disposable bag being equipped with a head plate (5) made of rigid material and having, on the one hand, an upwardly protruding inlet for a suction line and, on the other hand, an opening (8) which is sealed by a fluid-tight but gas-permeable membrane (9) and through which an underpressure can be generated in the inside of the bag, **characterized in that** the inlet for the suction line is at the same time designed as a securing device in order to secure the bag (B) from underneath onto a lid (2) of a rigid collection container (1), holding and coupling members in the form of resilient catches (7) being provided for coupling the head plate (5) of the bag (B) to the lid (2).

6. Disposable bag according to Claim 5, **characterized in that** the head plate (5) is provided with an additional passage which is equipped with a preferably breakable seal (11) and forms an air vent.

## Revendications

1. Dispositif de succion de fluides, notamment de fluides corporels dans le cadre d'opérations médicales, dans lequel le fluide est guidé au moyen d'une dépression par le biais d'une conduite de succion dans un récipient de collecte (B), le dispositif présentant le récipient de collecte (B) et un récipient extérieur rigide (1), le récipient de collecte (B) étant réalisé sous la forme d'un sachet à usage unique (B) installé dans le récipient extérieur (1), le sachet à usage unique (B) étant muni d'une partie de tête rigide (5) et la partie de tête rigide (5) présentant d'une part une entrée et d'autre part présentant une ouverture auxiliaire (8) fermée avec une membrane (9) perméable aux gaz, et au début de l'opération de succion, tout d'abord l'espace (A) entre la paroi extérieure du sachet et la paroi intérieure du récipient extérieur (1) étant évacué et ainsi la paroi flexible du sachet venant s'appliquer dans la direction de la paroi du récipient et seulement ensuite l'action de succion étant amorcée à travers la membrane (9) à l'intérieur du sachet et ainsi dans la conduite de succion,
**caractérisé en ce que**
en plus de la partie de tête rigide (5), il est prévu un couvercle (2) pouvant fermer de manière étanche à l'air le récipient extérieur (1), lequel couvercle est équipé, en plus d'une connexion (4) pour la conduite de succion, seulement d'une connexion (3) pour le raccordement à une source de succion, et
**en ce que** la partie de tête rigide (5) peut être connectée depuis le côté inférieur au couvercle du récipient (2) et est maintenue par ce dernier, l'entrée pouvant être accouplée de manière étanche à l'air à la connexion (4) pour la conduite de succion, et la connexion de raccordement (3) du couvercle (2) à la source de succion débouchant librement dans la portion du récipient extérieur (1) se trouvant au-dessus de la partie de tête rigide (5) et
**en ce que** des organes de fixation et d'accouplement en forme d'ergots d'encliquetage élastiques (7) sont prévus pour l'accouplement de la partie de tête (5) du sachet (B) au couvercle (2) du récipient, ces organes de fixation et d'accouplement entourant l'entrée.

2. Dispositif selon la revendication 1, **caractérisé en ce que** l'entrée de la conduite de succion est réalisée sur la partie de tête (5) du sachet (B) sous forme de raccord de connexion (6), qui est entouré par les ergots d'encliquetage (7) disposés concentriquement à celui-ci.

3. Dispositif selon la revendication 2, **caractérisé en ce que** les ergots d'encliquetage (7) font saillie vers le haut et **en ce qu'**entre la paroi extérieure du raccord et le côté intérieur des ergots d'encliquetage (7) on prévoit un espace annulaire ouvert vers le haut pour l'insertion de la conduite de succion.

4. Dispositif selon l'une quelconque des revendications 1 à 3, **caractérisé en ce que** dans la partie de tête rigide (5) du sachet (B) est prévu un passage supplémentaire (10) qui est pourvu d'une fermeture (11) de préférence pouvant être cassée afin de former une ouverture de désaérage pour vider le sachet.

5. Sachet à usage unique destiné à recevoir un fluide à aspirer et à être utilisé dans un dispositif selon l'une quelconque des revendications 1 à 4, étant muni d'une plaque de tête (5) en matériau rigide, une connexion saillant vers le haut étant d'une part prévue pour une conduite de succion et une ouverture (8) fermée avec une membrane (9) bloquant les fluides mais perméable aux gaz étant prévue d'autre part, à travers laquelle on peut créer à l'intérieur du sachet une dépression, **caractérisé en ce que** la connexion pour la conduite de succion est simultanément réalisée sous la forme d'un dispositif de fixation pour fixer le sachet (B) par son côté inférieur à un couvercle (2) d'un récipient de collecte rigide (1), des organes de fixation et d'accouplement en forme d'ergots d'encliquetage élastiques (7) étant prévus pour l'accouplement de la plaque de tête (5) du sachet (B) avec le couvercle (2).

6. Sachet à usage unique selon la revendication 5, **caractérisé en ce que** l'on prévoit dans la plaque de tête (5) un passage supplémentaire, formant une ouverture de désaérage, pourvu d'une fermeture (11) de préférence cassable.
